# EUROPÄISCHE PATENTANMELDUNG

(11) **EP 1 498 091 A1**
(43) Veröffentlichungstag der Anmeldung: **19.01.2005**
(21) Anmeldenummer: 04016131.7
(22) Anmeldetag: 08.07.2004
(51) Int. Cl.: A61F 5/00, A61M 25/00

(54) **Kit zur Katheterisation männlicher Personen**

(30) Priorität: 15.07.2003 DE 20310867 U
(71) Anmelder: Grundke, Reinhold, 84489 Burghausen (DE); Jaeniche, Wilhelm, 77694 Kehl-Leutesheim (DE); Liedl, Bernhard, Dr. med., 81337 München (DE); Götz, Alois H., Dr., 91257 Pegnitz (DE)
(72) Erfinder: Grundke, Reinhold, 84489 Burghausen (DE); Jaeniche, Wilhelm, 77694 Kehl-Leutesheim (DE); Liedl, Bernhard, Dr. med., 81337 München (DE); Götz, Alois H., Dr., 91257 Pegnitz (DE)
(74) Vertreter: Hano, Christian, Dipl.-Ing.

(57) **Zusammenfassung**

Der Kit zur Katheterisation männlicher Personen umfasst einen einteiligen Auffangkörper (10) aus Silikonkautschuk, der im mittleren Bereich einen Kammerabschnitt (12) aufweist, dessen Innenquerschnitt größer als der Querschnitt des Penis eines Benutzers ist, im vorderen Bereich einen an den zylindrischen Kammerabschnitt (12) anschließenden, sich nach vorne verjüngenden Trichterabschnitt (14) aufweist, der in einen zylindrischen Endabschnitt (16) übergeht, und im rückwärtigen Bereich einen an den Kammerabschnitt (12) anschließenden, sich nach hinten verjüngenden Übergangsabschnitt (18) aufweist, der in einen zylinderförmigen Abschlussabschnitt (20) übergeht, dessen Innendurchmesser etwas geringer ist als der Außendurchmesser des Penis des Benutzers. Ein schlauchförmiger Harnröhrenkatheter (32) mit einem Einlaufende (34) und einem Auslaufende (36) geht im Einsatz durch den Auffangkörper (10) hindurch, wobei das Einlaufende (34) in der Harnblase der männlichen Person anzuordnen ist. Mittels einer Klemmeinrichtung (16, 42) wird der Harnröhrenkatheter (32) im Einsatz zugfest und dicht an dem zylindrischen Abschnitt (16) gehalten.

## Beschreibung

Die Erfindung betrifft ein Kit zur Katheterisation männlicher Personen mit einem schlauchförmigen Harnröhrenkatheter.

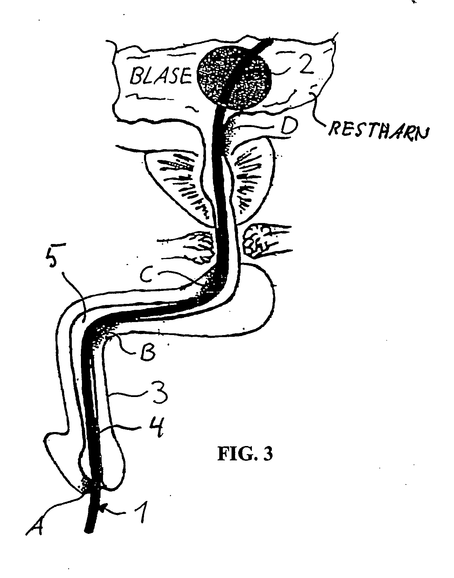

Zur Katheterisation männlicher Personen ist die Verwendung eines Ballondauerkatheters bekannt, wie sie in Fig. 3 gezeigt ist. Der Ballondauerkatheter 1 weist einen zweilumigen Katheterschlauch 4 auf, der durch die Harnröhre 5 eines Penis 3 in die Harnblase des männlichen Patienten eingeführt wird. An dem sich in der Harnblase befindlichen Einlaufende des Katheters 1 ist ein Ballon 2 vorgesehen, der über ein Lumen des zweilumigen Schlauches 4 aufgeblasen werden kann. Durch den Ballon 2 wird ein Herausrutschen des Katheterschlauches 4 nach seiner Installation verhindert. Über den anderen Lumen wird der Urin in der Harnblase nach außen abgeführt. Da der Katheterschlauch 4 zweilumig ausgebildet ist, ist er relativ dick. Da der Katheterschlauch 4 durch den Ballon 2 in der Harnblase gehalten wird, können an den in Fig. 3 gezeigten Stellen A, B, C und D an der Harnröhre Druckschäden und hierdurch Harnröhrenstrikturen auftreten. Dies wird in der Fachsprache als "Bow -Stringing- Effekt" bezeichnet.

Schließlich befindet sich die Einlauföffnung des Katheterschlauches 4 oberhalb des Ballons 2, so dass in der Blase ein Restharn verbleibt, der auf Dauer zu Vergiftungserscheinungen führen kann.

An der Mündung der Harnröhre (Stelle A) können Keime eintreten und rasch zwischen Katheteroberfläche und Harnröhre in die Harnblase hochwandern. Schließlich führt ein hydrostatischer Sog des Urins (statisch und dynamisch) zu polypoider Zystitis und zu Schmerzen.

Der Erfindung liegt die Aufgabe zugrunde, ein Kit zur Katheterisation männlicher Personen bereit zu stellen, das eine weitgehend schmerz- und keimfreie Katheterisation männlicher Personen auf zuverlässige Weise ermöglicht.

Diese Aufgabe wird durch ein Kit zur Katheterisation männlicher Personen mit einem einteiligen Auffangkörper und einem schlauchförmigen Katheter gemäß dem Patentanspruch 1 gelöst.

Vorteilhafte Weiterbildungen des erfindungsgemäßen Kits sind Gegenstand der Patentansprüche 2 und 3.

Das erfindungsgemäße Kit ermöglicht eine weitgehend schmerz- und keimfreie Katheterisation, da nach steriler Einlage des Harnröhrenkatheters und Anbringung des Auffangkörpers ein steriles geschlossenes Harnableitungssystem entsteht, wobei auch die Mündung sauber bleibt. Hierdurch wird die Gefahr einer nosokomialen, d. h. im Krankenhaus erworbenen Infektion reduziert.

Es ist möglich, einen einlumigen, dünnen, atoxischen Harnröhrenkatheter zu verwenden, wodurch die Reizung der Harnröhre vermindert wird. Da der Harnröhrenkatheter locker in die Harnröhre eingebracht werden kann, und keine Haltevorrichtung für den Harnröhrenkatheter innerhalb der Harnblase vorgesehen werden muss, entstehen keine Zug- und Druckschäden durch den "Bow-Stringing-Effect".

Vorzugsweise ist die Stirnseite des Einlaufendes des Harnröhrenkatheters geschlossen und wenigstens eine Durchgangsöffnung am Umfang des Einlaufendes des Harnröhrenkatheters vorgesehen. Die wenigstens eine Durchgangsöffnung kann angrenzend an den Blasenausgang positioniert werden, so dass kein Restharn in der Blase verbleibt.

Für eine einfache Einführung und zur Minimierung der Verletzungsgefahr ist die Stirnseite des Einlaufendes vorzugsweise abgerundet.

Ein Ausführungsbeispiel der Erfindung wird nachstehend anhand von Zeichnungen näher erläutert. Es zeigen:
- Fig. 1: in einer perspektivischen Ansicht schematisch die Anordnung eines Harnröhrenkatheters durch einen Auffangkörper zur Harnröhrenkatheterisation männlicher Personen, wobei ein während der Anordnung in den Auffangkörper eingeführter Penis nicht gezeigt ist;

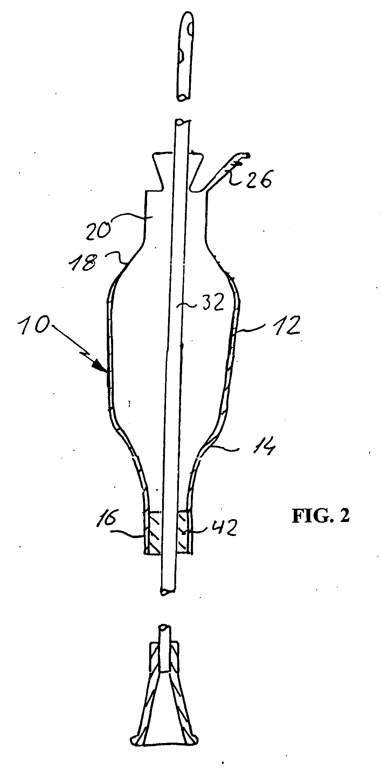

- Fig. 2: einen Längsschnitt durch den Auffangkörper von Fig. 1 bei Anordnung des des Harnröhrenkatheters durch den Auffangkörper;
- Fig. 3: die Anbringung eines bekannten Ballondauerkatheters

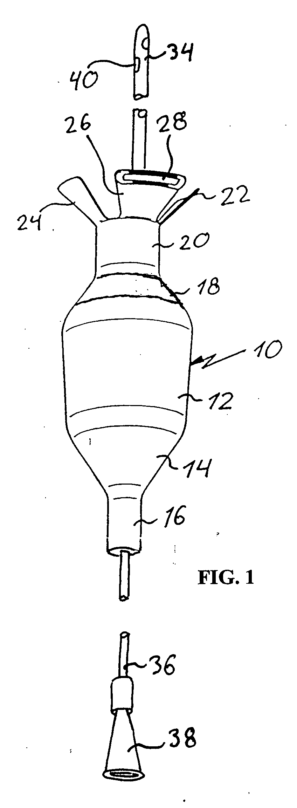

Der einteilige, rotationssymetrische Auffangkörper 10, der ein Teil eines Kits zur Katheterisation männlicher Personen bildet, besteht aus Silikonkautschuk und umfasst in seinem mittleren Bereich einen zylindrischen Kammerabschnitt 12, dessen Innendurchmesser größer als der Außendurchmesser des Penis eines Benutzers ist. An diesen Kammerabschnitt 12 schließt vorne (in Fig. 1 unten) ein sich nach vorne konisch verjüngender Trichterabschnitt 14 an, der an seinem vorderen Ende in einen Zylinderabschnitt 16 übergeht. Auf der dem Trichterabschnitt 14 entgegengesetzten Seite des Kammerabschnitts 12 geht der Kammerabschnitt 12 in einen kegelstumpfförmigen Übergangsabschnitt 18 über, der sich nach hinten (in Fig. 1 nach oben) konisch verjüngt. An diesen Übergangsabschnitt 18 schließt ein zylindrischer Abschlussabschnitt 20 an, dessen Innendurchmesser etwas geringer ist, als der Außendurchmesser des Penis eines Benutzers. Der Ausdruck "etwas geringer" ist so auszulegen, dass der Innendurchmesser z.B. bei einem Penisdurchmesser von 22 bis 40 mm ungefähr 20 mm beträgt. An dem rückwärtigen stirnseitigen Ende des Abschlussabschnittes 20 sind diametral gegenüberliegend zwei Dehnzungen 22, 24 vorgesehen. Zwischen den Dehnzungen 22, 24 ist eine Befestigungszunge 26 angeordnet, die angrenzend an ihr freies Ende eine Durchgangsöse 28 aufweist.

Der andere Teil des Kits zur Katheterisation männlicher Personen wird von einem schlauchförmigen einlumigen Harnröhrenkatheter 32 gebildet, dessen in die Blase einzuführendes Ende 34 an der Stirnseite abgerundet und verschlossen ist, um eine Einführung des Harnröhrenkatheters 32 zu erleichtern und die Verletzungsgefahr zu minimieren. Am Außenumfang des Endes 34 sind in Längsrichtung nacheinander mehrere Durchgangsöffungen 40 in Umfangsrichtung versetzt vorgesehen.

An dem anderen Ende des Harnröhrenkatheters 32 ist ein Schlauchanschluss 38 vorgesehen an den im Einsatz ein Ende eines Abführschlauches steril angeschlossen wird, dessen anderes Ende mit einem Beinbeutel (nicht gezeigt) zur Aufnahme von Urin in Verbindung steht

Zur Katheterisation wird der Harnröhrenkatheter 32 steril mit seinem Einlaufende 34 durch die Harnröhre bis in die Harnblase eingeführt, bis sich die Durchgangsöffnungen 40 in der Blase befinden. Der Durchmesser des Harnröhrenkatheters 32 ist so bemessen, dass der Harnröhrenkatheter 32 locker in der Harnröhre liegt.

Anschließend wird der Auffangkörper 10 an den Dehnzungen 22, 24 ergriffen und diese radial zur Mittelachse des Auffangkörpers 10 nach außen gezogen, wodurch sich der Innenquerschnitt des Abschlussabschnittes 20 so weit vergrößert, dass der Penis in den Auffangkörper 10 eingeführt werden kann. Anschließend werden die Dehnzungen 22, 24 losgelassen, wodurch sich der Abschlussabschnitt 20 an den Penis mit einer Druckkraft anschmiegt, die einen Austritt von Harn über den Abschlussabschnitt 20 verhindert. Gleichzeitig ist die Druckkraft so ausgelegt, dass kein Blutstau im Penis auftritt.

Dann wird die Vorrichtung mittels der Öse 28 an der Befestigungszunge 26 an einen Gürtel oder dgl. befestigt. Anstatt der Öse 28 kann auch ein Klettband oder dgl. an der Befestigungszunge 26 angebracht werden.

Auf dem Harnröhrenkatheter ist eine Hülse 42 aus Silikonkautschuk verschiebbar angebracht, wobei der Innendurchmesser der Hülse 42 so dimensioniert ist, dass eine hohe Haftreibung zwischen Harnröhrenkatheter 32 und Hülse 42 vorhanden ist. Die Hülse 42 wird auf dem Harnröhrenkatheter 42 so verschoben, dass sie in dem zylindrischen Abschnitt 16 angeordnet ist, wenn das Ende 34 des Harnröhrenkatheters 32 richtig in der Blase positioniert ist. Der Außendurchmesser der Hülse 42 ist etwas größer als der Innendurchmesser des zylindrischen Abschnitts 16, so dass bei Platzierung der Hülse 42 in dem zylindrischen Abschnitt 16, dieser gedehnt wird und eine zusätzliche Klemmkraft auf den Harnröhrenkatheter 32 ausübt, die im Einsatz ein Verrutschen des Harnröhrenkatheters 32 sicher verhindert. Außerdem wird hierdurch ein luftdichter Abschluss geschaffen, der ein Eindringen von Keimen und Bakterien verhindert. Eine Haltevorrichtung für den Harnröhrenkatheter 32 innerhalb der Harnblase ist somit entbehrlich.

Dies ist nur eine Möglichkeit zur Ausbildung einer Klemmeinrichtung, mit der der Harnröhrenkatheter 32 an dem zylindrischen Abschnitt 16 zugfest angebracht werden kann. Es sind auch andere Variationen denkbar. Beispielsweise kann der Harnröhrenkatheter in dem zylindrischen Abschnitt angeordnet und eine Hülse mit ausreichender Klemmkraft auf den zylindrischen Abschnitt aufgeschoben werden.

Die Dimensionen des Auffangkörpers 10 können abhängig von der jeweiligen Größe des Penis eines Benutzers ausgelegt werden. Bei einem für den durchschnittlichen Benutzer ausgelegten Auffangkörper 10 beträgt der Innendurchmesser des Kammerabschnitts ungefähr 43 mm. Der Abschlussabschnitt 20 dieser Ausführungsform hat bevorzugt einen Innendurchmesser von 20 mm und einen Außendurchmesser von 21 mm. Die Länge des Abschlussabschnittes 20 beträgt ca. 17,5 mm. Eine solche Ausführungsform ist für einen Penisdurchmesser zwischen 22 und 40 mm geeignet.

Nach der sterilen Einlage des Harnröhrenkatheters 32 und der Anbringung des Auffangkörpers 10 besteht ein geschlossenes Harnableitungssystem, wobei zunächst auch die Mündung der Harnröhre sauber bleibt Die Gefahr einer nosokomialen, d.h. im Krankenhaus erworbenen Infektion wird somit weitgehend verhindert.

Schließlich ist es möglich einlumige, dünne Harnröhrenkatheter zu verwenden, die die Reizung der Harnröhre verhindern.

## Patentansprüche

1. Kit zur Katheterisation männlicher Personen mit einem einteiligen Auffangkörper (10) aus Silikonkautschuk, der
- im mittleren Bereich einen Kammerabschnitt (12) aufweist, dessen Innenquerschnitt größer als der Querschnitt des Penis eines Benutzers ist,
- im vorderen Bereich einen an den zylindrischen Kammerabschnitt (12) anschließenden, sich nach vorne verjüngenden Trichterabschnitt (14) aufweist, der in einen zylindrischen Endabschnitt (16) übergeht, und
- im rückwärtigen Bereich einen an den Kammerabschnitt (12) anschließenden, sich nach hinten verjüngenden Übergangsabschnitt (18) aufweist, der in einen zylinderförmigen Abschlussabschnitt (20) übergeht, dessen Innendurchmesser etwas geringer ist als der Außendurchmesser des Penis des Benutzers,
mit einem schlauchförmigen Harnröhrenkatheter (32) mit einem Einlaufende (34) und einem Auslaufende (36), der im Einsatz durch den Auffangkörper (10) hindurchgeht, wobei das Einlaufende (34) in der Harnblase der männlichen Person anzuordnen ist, und mit einer Klemmeinrichtung (16, 42) mit der der Harnröhrenkatheter (32) im Einsatz zugfest und dicht an dem zylindrischen Abschnitt (16) gehalten wird.

2. Kit nach Anspruch 1, **dadurch gekennzeichnet, dass** die Stirnseite des Einlaufendes (34) des Harnröhrenkatheters (32) geschlossen ist, und wenigstens eine Durchgangsöffnung (40) am Umfang des Einlaufendes (34) des Harnröhrenkatheters (32) vorgesehen ist.

3. Kit nach Anspruch 2, **dadurch gekennzeichnet, dass** die Stirnseite des Einlaufendes (34) des Harnröhrenkatheters (32) abgerundet ist.
